# EUROPEAN PATENT APPLICATION

(11) **EP 2 062 885 A1**
(43) Date of publication of application: **27.05.2009**
(21) Application number: 07121221.1
(22) Date of filing: 21.11.2007
(51) Int. Cl.: C07D 401/04, A61K 31/505, A61P 35/00

(54) **Acid addition salts of imatinib and formulations comprising the same**

(71) Applicant: Eczacibasi-Zentiva Kimyasal Ürünler Sanayi ve Ticaret A.S., Istanbul 34394 (TR)
(72) Inventor: Adiyaman, Mustafa, Fatih cad. No:12 Cerkezköy 59500, Tekirdag (TR); Durmus, Semih, Fatih cad. No:12 Cerkezköy 59500, Tekirdag (TR); Sahpaz Cerit, Filiz, Fatih cad. No:12 Cerkezköy 59500, Tekirdag (TR)
(74) Representative: Sevinç, Erkan

(57) **Abstract**

Acid addition salts of 4-[(4-Methyl-1-piperazinyl)methyl]-N-[4-methyl-3-[[4-(3-pyridinyl)-2-pyrimidinyl]amino]phenyl]-benzamide selected from the group consisting of an orotate salt, an oxalate salt, a nicotinate salt, a camphor sulfonate salt, a paratoluene sulfonate salt, a laurate salt, an aspartate salt, a caprate salt, an oleate salt and a phenyl acetate salt is disclosed. The invention further provides a method for producing said acid addition salts and formulations, preferably solid formulations comprising at least one of said salts within the composition.

## Description

### Technical Field of the Invention

The present invention relates to new salts of 4-[(4-Methyl-1-piperazinyl) methyl]-N-[4-methyl-3-[[4-(3-pyridinyl)-2-pyrimidinyl]amino]phenyl]-benzamide (Imatinib), particularly new acid addition salts thereof which are considerably non-hygroscopic and less toxic compared to the known salts in the art.

### Background of the Invention

4-[(4-Methyl-1-piperazinyl)methyl]-N-[4-methyl-3-[[4-(3-pyridinyl)-2-pyrimidinyl] amino]phenyl]-benzamide, hereinafter referred with its generic commercial name Imatinib, and its pharmaceutically acceptable salts are used as anti-tumor agents for treating certain types of leukemia and other cancers of the blood cells. They are also used in the treatment of gastrointestinal stromal tumor and a number of other malignancies.

Imatinib free base was introduced by EP 564 409 and various salts thereof have been disclosed in numerous publications. International Publication No. WO 2004/074502 discloses preparation of acid addition salts, particularly hydrated or anhydrous halogenated salts of imatinib. Many of acid addition salts of imatinib free base were also described for instance in WO 2005/075454. This international application discloses tartrate salt, hydrochloride salt, citrate salt, malate salt, fumarate salt, succinate salt, benzoate salt, benzenesulfonate salt, pamoate salt, formate salt, malonate salt, 1, 5-naphthalenedisulfonate salt, salicylate salt, acyclohexane sulfamate salt, lactate salt, mandelate salt, glutarate salt, adipate salt, squarate salt, vanillate salt, oxaloacetate salt, ascorbate salt, and sulfate salt of imatinib. These salts are reported to have increased water solubility compared to the free base form. However, the salts listed above are designed for oral administration and are desired to have increased solubility when used in a solution form. In other words, highly hygroscopic nature of this salt group renders them inappropriate for tablet form applications or requires them to be coated effectively for such administration routes.

Mesylate (methane sulfonic acid) salt of the imatinib used as a pharmaceutical active ingredient has been the most popular one among all the know salts and has been also subjected to many improvements in terms of morphology and formulation. Various crystal forms of imatinib mesylate have been disclosed for instance in WO 99/03854, WO 2006/024863, WO 2006/048890, WO 2005/077933, WO 2006/054314, WO 2004/106326 and WO 2005/095379. Such improvements in crystallinity of the mesylate salt have been directed to eliminate its unstable nature. However, all these efforts for obtaining stable polymorphs do not render the mesylate salts of imatinib substantially non-toxic and sufficiently safe for high dosages. Methane sulfonic acid is known with its highly poisonous nature and also its imatinib salt is known to cause side effects in some cases which can well be attributed to its toxic effects. Furthermore, certain crystal forms of imatinib mesylate (e.g. α crystal form) are characterized by needle-shaped crystals and hygroscopic nature as per described in the Intl. Publ. No. WO 99/03854 and many other references.

Therefore, there is still a need for providing alternative salts of imatinib which are considerably less toxic with a reduced hygroscopic nature for use generally in solid dosage forms and sustained release formulations compared to the known salts available in the art as outlined above. The present invention solves the problems addressed with reference to the prior publications by providing the acid addition salts listed in claim 1.

### Objects of the Invention

The present invention solves the above problems by providing novel acid addition salts achieving the following objects, wherein;

A first object of the present invention is to provide alternative acid addition salts of imatinib active compound which are substantially less toxic compared to the known salts in the art,

Another object of the present invention is to provide alternative acid addition salts of imatinib active compound which have considerably lower hygroscopic nature and good flow properties,

A further object of the present invention is to provide alternative acid addition salts of imatinib suitable for use in solid dosage forms and sustained release formulations, particularly for use in tablet forms,

Still a further object of the present invention is to provide a process suitable for the preparation of novel acid addition salts of the present invention,

Yet another object of the present invention is to provide formulations comprising the salts, particularly solid administration forms of the acid addition salts according to the present invention.

### Detailed Description of the Invention

The synthesis of pharmaceutically acceptable acid addition salts of imatinib is generally conducted by the reaction of imatinib free base having the general formula (I) with a solution of an organic or inorganic acid in an organic or inorganic or a mixture thereof.

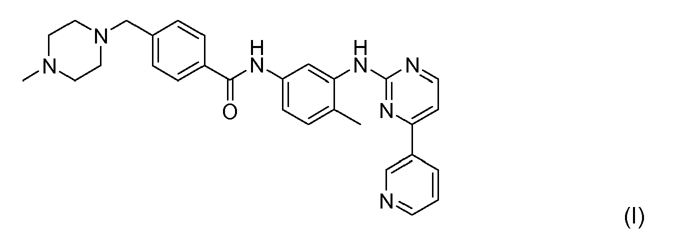

Apparently, a free base serves as an active ingredient and plenty of effort is spent for improving drug features, e.g. toxicity, hygroscopy, flow properties, stability, solubility etc. Prior art imatinib salts, particularly their acid part bear the well known drawbacks in terms of toxicity which renders those compounds unsuitable for use as an anti-tumor agent as expressed in the above explanations. The known compounds further carry the disadvantage in terms of hygroscopy which affect their stability and bioavailability, and makes them unsuitable for application routes other than solutions, and aqueous or non-aqueous suspension forms.

The present invention introduces a novel imatinib acid addition salt group which group namely consists of orotate, oxalate, nicotinate, camphor sulfonate, para-toluene sulfonate, laurate, aspartate, caprate, oleate and phenyl acetate salts. These salts are expected to be substantially less toxic compared to the conventional salts, particularly less than mesylate salt of imatinib, as the acids forming the listed group of salts are known with their considerably lower toxic nature.

In the following description, the acids forming the novel salts indicated herewith will be compared with the methane sulfonic acid which is used for obtaining wide spread imatinib mesylate. In the context of the present invention the term "LD₅₀" refers to index of toxicity, i.e. the amount of the substance that kills 50% of the test population of experimental animals when administered as a single dose. LD₅₀ data for methane sulfonic acid and acids of the present invention are given below for comparison.

**Table 1. LD₅₀ values of acids used for obtaining particular salts of imatinib**

| **Compound Name** | **Administration Route** | **Experimental Animal** | **LD₅₀ (mg/kg)** |
|---|---|---|---|
| Methanesulfonic | Oral | Quail | 1000 |
| acid | Oral | Rat | 200 |
| Orotic acid | Oral | Mouse | 2000 |
| | Intraperitoneal | Mouse | 841 |
| | Intravenous | Mouse | 770 |
| Oxalic Acid | Oral | Rat | 7500 |
| Nicotinic acid | Oral | Rat | 7000 |
| | Intraperitoneal | Mouse | 358 |
| | Intravenous | Mouse | 5000 |
| Camphor sulfonic acid | Subcutaneous | Mouse | 2502 |
| Lauric acid | Oral | Rat | 12000 |
| | Intravenous | Mouse | 131 |
| Aspartic acid | Oral | Rat | 5000 |
| | Skin | Rabbit | 5000 |
| p-Toluene sulfonic | Oral | Rat | 2480 |
| acid | Oral | Mouse | 400 |
| Capric acid | Oral | Rat | 3730 |
| | Oral | Rabbit | 1770 |
| Oleic acid | Oral | Rat | 74000 |
| | Intraperitoneal | Mouse | 282 |
| | Intravenous | Mouse | 230 |
| Phenyl acetic acid | Oral | Rat | 2250 |
| | Intraperitoneal | Rat | 1600 |

LD₅₀ data of the acids indicated in Table 1 are available at the internet web sites http://www.physchem.ox.ac.uk/MSDS, http://avogadro.chem.iastate.edu/MSDS, http://www.sloss.com/chemical/msds, http://www.sciencelab.com and in the reference book "Dangerous Properties of Industrial Materials", Van Nostrand Reinhold Press, 7th Edition, New York 1989, Volumes II and III.

LD₅₀ indexes of the acids used for obtaining the salts of the present invention are apparently much higher than that of the methanesulfonic acid, which in turn renders the acids of the invention considerably less toxic. Toxicity levels of oleic acid, lauric acid, oxalic acid and nicotinic acid are particularly and surprisingly lower in the test rats. Toxicity data of these and other acids used in the present invention implicitly indicate that such acids may well reduce the health risks of the conventional acids and reduce also possible unwanted effects after administration of the active ingredient. Another advantage of the acids used in the present invention will obviously appear in the production procedure of the active ingredient for the sake of safe production both for environment and also for the people interfering said production procedures.

The inventors also surprisingly found that imatinib salt forms of "orotic acid" and "oxalic acid" from the list of acids used in the present invention further provide the advantages of exhibiting considerable low hygroscopy and good flow properties which make them suitable for use in solid dosage forms, particularly in capsule or tablet forms. Imatinib orotate has the following general formula (II):

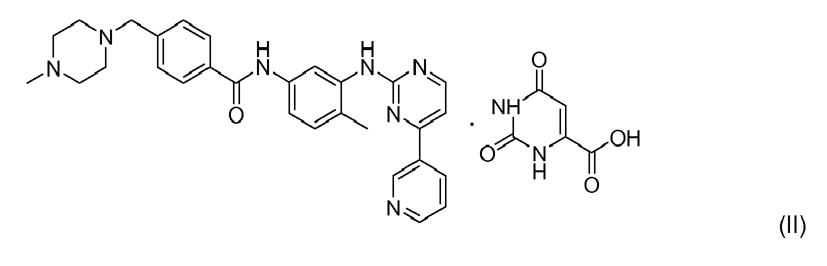

According to an embodiment of the presented invention one of the substantially non-toxic and non-hygroscopic salts outlined above, for instance imatinib orotate, is prepared by reacting imatinib free base (I) with orotic acid in the presence of an organic or inorganic solvent, or mixtures thereof. A preferred solvent that may be used in the preparation of imatinib orotate as well as other salts of the present invention is selected from the group consisting of methanol, ethanol, isopropanol, ethyl acetate, hexane, heptane, acetonitrile, dichloromethane, chloroform, water and mixtures thereof.

Orotic acid, having considerably lower LD₅₀ compared to the methane sulfonic acid as shown in Table 1, will obviously facilitate for providing a less toxic and less hygroscopic acid addition salt of imatinib active compound.

According to another embodiment of the presented invention imatinib oxalate having the general formula of (III) and having the advantages similar to that of imatinib orotate, is prepared by reacting imatinib free base (I) with oxalic acid in the presence of an organic or inorganic solvent, or mixtures thereof.

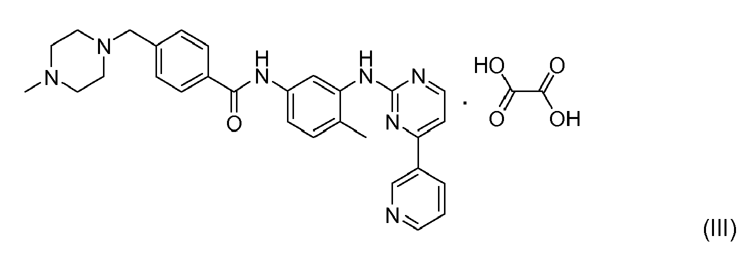

To this end, orotate and oxalate salts of imatinib have also good flow properties due to low hygroscopicity. For illustration of non-hygroscopic nature of the imatinib orotate and oxalate salts, they were left for 24 hours inside stability chambers under the conditions listed below (Table 2). The water contents were measured by a well known method, namely Karl Fischer method for determining water quantities.

**Table 2. Non-hygroscopicity test results**

| **Sample** | **Initial Moisture Content (%)** | **Final Moisture Content After 24hrs (%)** 25°C at humidity of 60% | **Final Moisture Content After 24hrs (%)** 40 °C at humidity of 75% |
|---|---|---|---|
| Imatinib Orotate | 0.4 | 0.7 | 0.7 |
| Imatinib Oxalate | 1.6 | 3.4 | 3.6 |

The inventors report that imatinib orotate and imatinib oxalate have shown good hygroscopic properties among other imatinib salts of the present invention. Orotate
and oxalate salts of the invention can also be regarded as considerably low hygroscopic and comparable to the α and β forms of imatinib mesylate available in the art (see Hygroscopicity test results of α and β forms of imatinib mesylate, WO 99/03854 A1; page 8).

Accordingly, in light of the above findings, it may be concluded that all the acid addition salts of the present invention have at least better features in terms of toxicity and particular salts *inter alia* (i.e. imatinib orotate and imatinib oxalate) have further advantages in terms of hygroscopicity.

According to a further embodiment, compositions comprising acid addition salts of the present invention are also presented herewith for illustrating the best mode of carrying out invention for persons skilled in the art. The acid salts of the invention may apparently be contained in combination with certain components such as binders, fillers, diluents, disintegrants, lubricants and coating materials according to the desired administration route. Solid oral dosage forms, e.g. hard gelatin capsules and coated tablets, are particularly preferred. The amount of the salts of the present invention ranges from 5% to 85%, more preferably 25% to 35% by weight.

The diluents that may be incorporated with a preferred formulation of the invention are preferably selected from the group of natural or artificial carbohydrates such as lactose, sucrose, glucose, starches, sorbitol and microcrystalline cellulose. The binders are similarly and preferably selected from the group of starches, sugars, cellulose, cellulose derivatives, gelatin and polyvinyl pyrrolidone. PVPPXL^{™}, a special commercial polyvinyl pyrrolidone is specifically preferred.

Starch and microcrystalline cellulose are also well established disintegrant materials for use in a preferred formulation of the present invention. Avicel^{™}, a type of commercial microcrystalline cellulose is particularly preferred.

Preferred lubricants for use in particular solid formulations are selected from the group consisting of talc, silica, magnesium stearate, glidan, aerosil, stearic acid and stearin. Magnesium stearate is particularly preferred.

Following examples are given solely for the purpose of illustration with no exclusion to show realization of the particular embodiments.

### Example 1

### Preparation of 4-[(4-Methyl-1-piperazinyl)methyl]-N-[4-methyl-3-[[4-(3-pyridinyl)-2-pyrimidinyl]amino]phenyl]-benzamide, orotate

The solution of 4-[(4-Methyl-1-piperazinyl)methyl]-N-[4-methyl-3-[[4-(3-pyridinyl)-2-pyrimidinyl]amino]phenyl]-benzamide (0.251 g, 0.50 mmol) and orotic acid (0.087 g, 0.50 mmol) in 1:1 water/ethanol mixture was stirred at 45°C. Upon cooling, the product was filtered and dried to afford 4-[(4-Methyl-1-piperazinyl)methyl]-N-[4-methyl-3-[[4-(3-pyridinyl)-2-pyrimidinyl]amino] phenyl]-benzamide, orotate. Smp by DSC: 259°C and 271°C (start of melting). ¹H NMR (300 MHz, DMSO-*d₆*): δ 11.03 (bs), 10.22 (s), 9.26 (bs), 9.02 (s), 8.67 (bs), 8.49 (t, *J* = 5.1 Hz ), 8.45 (bs), 8.07 (s), 7.93 (t, *J* = 7.8 Hz ), 7.44 (m), 7.20 (d, *J* = 8.1 Hz), 5.77 (s), 3.62 (s), 2.71 (s), 2.21 (s). ¹³C NMR (75 MHz, DMSO-*d6*): δ 165.3,165.2,161.7,161.2,159.6, 151.5, 151.0, 148.3, 137.9, 137.2, 134.6, 134.1, 130.2, 128.9, 127.8, 117.3, 116.9, 107.6, 99.6, 60.6, 52.7, 49.6, 42.5, 17.8. ESI-MS (m/z): Calcd for C₂₉H₃₂N₇O [M+H-C₅H₄N₂O₄]⁺: 494.3, Found 494.2. IR (cm⁻¹): 3262, 3035, 2808, 2620, 1692, 1681, 1658, 1639, 1534, 1494, 1483, 1454, 1411, 1359, 1313, 1286, 1249, 1130, 984, 849, 771, 748, 540. Anal. calcd for C₃₄H₃₅N₉O₅ + 0.2H₂O: C, 62.51; H, 5.42; N, 19.30. Found: C, 62.12; H, 5.35; N, 19.25.

### Example 2

### Preparation of 4-[(4-Methyl-1-piperazinyl)methyl]-N-[4-methyl-3-[[4-(3-pyridinyl) -2-pyrimidinyl]amino]phenyl]-benzamide, oxalate

4-[(4-Methyl-1-piperazinyl)methyl]-N-[4-methyl-3-[[4-(3-pyridinyl)-2-pyrimidinyl]amino] phenyl]-benzamide (0.251 g, 0.50 mmol) was added to the solution of oxalic acid (0.064 g, 0.50 mmol) in isopropanol. After filtration, a pale yellow solid was obtained.

Smp by DSC: 221°C (start of melting). IR (cm⁻¹): 3437, 3024, 1655, 1581, 1523, 1408, 1383, 1313, 1269, 1192, 1018, 954, 870, 808, 754, 706, 571. Anal. calcd for C₃₁H₃₃N₇O₅+ 0.2C₃H₈O: C, 63.70; H, 5.81; N, 16.46. Found: C, 63.83; H, 5.84; N, 16.29.

### Example 3

### Preparation of 4-[(4-Methyl-1-piperazinyl)methyl]-N-[4-methyl-3-[[4-(3-pyridinyl) -2-pyrimidinyl]amino]phenyl]-benzamide, nicotinate

A solution of 4-[(4-Methyl-1-piperazinyl)methyl]-N-[4-methyl-3-[[4-(3-pyridinyl)-2-pyrimidinyl]amino]phenyl]-benzamide (0.256 g, 0.52 mmol) with nicotinic acid (0.064 g, 0.52 mmol) in hot ethanol was stirred for 1-2 hours. A yellow oil was obtained after the removal of volatiles *in vacuo*. The resulting residue was washed with acetone for 2-4 h. A yellow solid was obtained after the removal of volatiles *in vacuo*.

MP: 76-78°C. IR (cm⁻¹): 3389, 2833, 1663, 1660, 1599, 1555, 1534, 1478, 1450, 1420, 1406, 1178, 1127, 986, 836, 806, 758.

### Example 4

### Preparation of 4-[(4-Methyl-1-piperazinyl)methyl]-N-[4-methyl-3-[[4-(3-pyridinyl)-2-pyrimidinyl]amino]phenyl]-benzamide, camphor sulfonate

A solution of 4-[(4-Methyl-1-piperazinyl)methyl]-N-[4-methyl-3-[[4-(3-pyridinyl)-2-pyrimidinyl]amino]phenyl]-benzamide (0.250 g, 0.50 mmol) and camphor sulfonic acid (0.116 g, 0.50 mmol) in water was stirred at RT for 1-3 h. The solvent was evaporated *in vacuo* at 40-50°C. The residue was dissolved in 2 mL of dichloromethane forming yellow solution. The solution was slurried with cold hexane. The product was filtered and dried to yield 4-[(4-Methyl-1-piperazinyl)methyl]-N-[4-methyl-3-[[4-(3-pyridinyl)-2-pyrimidinyl] amino]phenyl]-benzamide, camphor sulfonate.

MP: 120-123°C. IR (cm⁻¹): 3423, 2957, 1739, 1654, 1479, 1452, 1418, 1288, 1188, 1128, 1042, 1027, 984, 802, 709, 692, 618, 584.

### Example 5

### Preparation of 4-[(4-Methyl-1-piperazinyl)methyl]-N-[4-methyl-3-[[4-(3-pyridinyl) -2-pyrimidinyl]amino]phenyl]-benzamide, para-toluene sulfonate

4-[(4-Methyl-1-piperazinyl)methyl]-N-[4-methyl-3-[[4-(3-pyridinyl)-2-pyrimidinyl] amino] phenyl]-benzamide (0.250 g, 0.50 mmol) was added to the solution of p-toluene sulfonic acid (0.087 g, 0.50 mmol) in ethanol. The reaction mixture was stirred at 40-50°C for 1-2 h. The volatiles were removed *in vacuo* until one-third of the reaction mixture left. After the addition of cold hexane, the solution was cooled in an ice bath. The product was isolated following the filtration as a yellow solid

### Example 6

A capsule form comprising one or more of the acid addition salts of the present invention is incorporated with preferred auxiliary materials to result the following composition having the indicated dosage form.

**Table 3. Pharmaceutical composition containing imatinib salts of the present invention as an active ingredient.**

| **No** | **Material** | **Amount (mg)** |
|---|---|---|
| 1 | Salt | 100 |
| 2 | Avicel | 200 |
| 3 | PVPPXL | 15 |
| 4 | Aeorosil | 2 |
| 5 | Magnesium stearate | 1.5 |
| | Average weight | 318.5 |

## Claims

**1.** An acid addition salt of 4-[(4-Methyl-1-piperazinyl)methyl]-N-[4-methyl-3-[[4-(3-pyridinyl)-2-pyrimidinyl]amino]phenyl]-benzamide selected from the group consisting of an orotate salt, an oxalate salt, a nicotinate salt, a camphor sulfonate salt, a para-toluene sulfonate salt, a laurate salt, an aspartate salt, a caprate salt, an oleate salt and a phenyl acetate salt.

**2.** A process for producing acid addition salts of 4-[(4-Methyl-1-piperazinyl) methyl]-N-[4-methyl-3-[[4-(3-pyridinyl)-2-pyrimidinyl]amino]phenyl]-benzamide comprising;
reacting 4-[(4-Methyl-1-piperazinyl) methyl]-N-[4-methyl-3-[[4-(3-pyridinyl)-2-pyrimidinyl]amino]phenyl]-benzamide with an acid selected from the group consisting of orotic acid, oxalic acid, nicotinic acid, camphor sulfonic acid, para-toluene sulfonic acid, lauric acid, aspartic acid, capric acid, oleic acid, and phenyl acetic acid.

**3.** The process of claim 1 wherein the reaction is carried out in the presence of an organic or inorganic solvent, or a mixture thereof.

**4.** The process of claim 3 wherein the solvent is selected from the group consisting of methanol, ethanol, isopropanol, ethyl acetate, hexane, heptane, acetonitrile, dichloromethane, chloroform and water.

**5.** Use of an acid addition salt of imatinib, selected from the group consisting of imatinib orotate, imatinib oxalate, imatinib nicotinate, imatinib camphor sulfonate, imatinib para-toluene sulfonate, imatinib laurate, imatinib aspartate, imatinib caprate, imatinib oleate and imatinib phenyl acetate in the preparation of a medicament for treatment of cancer.

**6.** A formulation comprising at least one of the acid addition salts of claim 1.

**7.** A formulation according to claim 6 wherein said formulation is in solid oral dosage form.

**8.** A formulation according to claim 7 wherein said formulation is in tablet or capsule form.

**9.** A formulation according to claim 7 wherein the acid addition salt is selected from the group of imatinib orotate and imatinib oxalate.

**11.** A formulation according to claim 6 wherein the formulation further comprises diluents selected from the group of lactose, sucrose, glucose, starches, sorbitol and microcrystalline cellulose.

**12.** A formulation according to claim 6 wherein the formulation further comprises binders that are selected from the group of starches, sugars, cellulose, cellulose derivatives, gelatin and polyvinyl pyrrolidone.

**13.** A formulation according to claim 6 wherein the formulation further comprises disintegrant materials selected from the group of starches and microcrystalline cellulose.

**14.** A formulation according to claim 6 wherein the formulation further comprises lubricants selected from the group consisting of talc, silica, magnesium stearate, glidan, aerosil, stearic acid and stearin.
